(19) Europäisches Patentamt
European Patent Office
Office européen des brevets

(11) **EP 2 723 888 B1**

(12) **EUROPEAN PATENT SPECIFICATION**

(45) Date of publication and mention
of the grant of the patent:
**11.01.2017 Bulletin 2017/02**

(51) Int Cl.:
***C12Q 1/68*** (2006.01)

(21) Application number: **11868033.9**

(86) International application number:
**PCT/CN2011/076187**

(22) Date of filing: **23.06.2011**

(87) International publication number:
**WO 2012/174723 (27.12.2012 Gazette 2012/52)**

(54) **RISK ASSESSMENT FOR PHENYTOIN-INDUCED ADVERSE DRUG REACTIONS**

RISIKOBEURTEILUNG FÜR PHENYTOIN-INDUZIERTE ARZNEIMITTELNEBENWIRKUNGEN

ÉVALUATION DE RISQUE DE RÉACTIONS INDÉSIRABLES AUX MÉDICAMENTS INDUITES PAR LA PHÉNYTOÏNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**30.04.2014 Bulletin 2014/18**

(73) Proprietor: **Chang Gung Medical Foundation Chang Gung**
**Memorial Hospital at Keelung**
**Keelung City 20401 (TW)**

(72) Inventors:
• **CHUNG, Wen-Hung**
**Guishan Township**
**Taoyuan County 333 (TW)**
• **HUNG, Shuen-Iu**
**Guishan Township**
**Taoyuan County 333 (TW)**

(74) Representative: **Lang, Christian**
**LangPatent Anwaltskanzlei**
**IP Law Firm**
**Rosenheimer Straße 139**
**81671 München (DE)**

(56) References cited:
**WO-A1-2005/047544     US-A1- 2001 034 023**
**US-A1- 2006 269 935**

• **KUDZI WILLIAM ET AL: "Characterisation of CYP2C8, CYP2C9 and CYP2C19 polymorphisms in a Ghanaian population", BMC MEDICAL GENETICS, BIOMED CENTRAL, LONDON, GB, vol. 10, no. 1, 2 December 2009 (2009-12-02), page 124, XP021066767, ISSN: 1471-2350**
• **ROBERT T. KINOBE ET AL: "P450 2C18 Catalyzes the Metabolic Bioactivation of Phenytoin", CHEMICAL RESEARCH IN TOXICOLOGY, vol. 18, no. 12, 1 December 2005 (2005-12-01), pages 1868-1875, XP055145974, ISSN: 0893-228X, DOI: 10.1021/tx050181o**
• **LÓPEZ MARISOL ET AL: "Pharmacogenetics of the antiepileptic drugs phenytoin and lamotrigine.", DRUG METABOLISM AND DRUG INTERACTIONS 2011, vol. 26, no. 1, May 2011 (2011-05), pages 5-12, XP009180683, ISSN: 0792-5077**
• **JUNJI SARUWATARI ET AL: "Update on the genetic polymorphisms of drug-metabolizing enzymes in antiepileptic drug therapy", PHARMACEUTICALS, M D P I AG, CH, vol. 3, no. 8, 20 August 2010 (2010-08-20) , pages 2709-2732, XP002702943, ISSN: 1424-8247, DOI: 10.3390/PH3082709**
• **GOLDSTEIN J A: "Clinical relevance of genetic polymorphisms in the human CYP2C subfamily", BRITISH JOURNAL OF CLINICAL PHARMACOLOGY, BLACKWELL SCIENTIFIC PUBL, GB, vol. 52, no. 4, 1 October 2001 (2001-10-01), pages 349-355, XP001121634, ISSN: 0306-5251, DOI: 10.1046/J.0306-5251.2001.01499.X**

**(Cont. next page)**

- LÖSCHER WOLFGANG ET AL: "The clinical impact of pharmacogenetics on the treatment of epilepsy", EPILEPSIA, vol. 50, no. 1, 1 January 2009 (2009-01-01), pages 1-23, XP055145800, ISSN: 0013-9580, DOI: 10.1111/j.1528-1167.2008.01716.x
- KLOTZ ULRICH: "The role of pharmacogenetics in the metabolism of antiepileptic drugs - Pharmacokinetic and therapeutic implications", CLINICAL PHARMACOKINETICS, vol. 46, no. 4, 2007, pages 271-279, XP009180697, ISSN: 0312-5963
- BRANDOLESE ET AL: "Severe phenytoin intoxication in a subject homozygous forCYP2C9*3", CLINICAL PHARMACOLOGY AND THERAPEUTICS, NATURE PUBLISHING GROUP, US, vol. 70, no. 4, 1 October 2001 (2001-10-01), pages 391-394, XP005743723, ISSN: 0009-9236
- LEE AI-YOUNG, KIM MIN-JUNG, CHEY WON-YOUNG, CHOI JUN, KIM BYUNG-GUN: "Genetic polymorphism of cytochrome P 450 2C9 in diphenylhydantoin-induced cutaneous adverse drug reactions", EUROPEAN JOURNAL OF CLINICAL PHARMACOLOGY, vol. 60, no. 3, 1 May 2004 (2004-05-01), pages 155-159, XP055146123, ISSN: 0031-6970, DOI: 10.1007/s00228-004-0753-0
- GOLDSTEIN J A ET AL: "EVIDENCE THAT CYP2C19 IS THE MAJOR (S)-MEPHENYTOIN 4'-HYDROXYLASE IN HUMANS", BIOCHEMISTRY, AMERICAN CHEMICAL SOCIETY, US, vol. 33, no. 7, 1 July 1994 (1994-07-01), pages 1743-1752, XP000560259, ISSN: 0006-2960, DOI: 10.1021/BI00173A017
- CELESTE B.L. MAN ET AL: "Association between HLA-B*1502 Allele and Antiepileptic Drug-Induced Cutaneous Reactions in Han Chinese", EPILEPSIA, vol. 48, no. 5, 1 May 2007 (2007-05-01), pages 1015-1018, XP055176743, ISSN: 0013-9580, DOI: 10.1111/j.1528-1167.2007.01022.x
- FDA: "Postmarket Drug Safety Information for Patients and Providers > Phenytoin and Fosphenytoin Information", Postmarket Drug Safety Information for Patients and Providers, 24 November 2008 (2008-11-24), pages 1-2, XP055176731, Retrieved from the Internet: URL:http://www.fda.gov/Drugs/DrugSafety/PostmarketDrugSafetyInformationforPatientsandProviders/ucm110259.htm [retrieved on 2015-03-16]
- FERRELL P B ET AL: "Carbamazepine, HLA-B*1502 and risk of Stevens-Johnson syndrome and toxic epidermal necrolysis: US FDA recommendations", PHARMACOGENOMICS, vol. 9, no. 10, 1 October 2008 (2008-10-01), pages 1543-1546, XP055176763, ISSN: 1462-2416, DOI: 10.2217/14622416.9.10.1543
- MARK MCCORMACK ET AL: "Genome-wide mapping for clinically relevant predictors of lamotrigine- and phenytoin-induced hypersensitivity reactions", PHARMACOGENOMICS, vol. 13, no. 4, 1 March 2012 (2012-03-01), pages 399-405, XP055176757, ISSN: 1462-2416, DOI: 10.2217/pgs.11.165
- WILLIAM KUDZI ET AL.: 'Characterisation of CYP2C8, CYP2C9 and CYP2C19 polymorphisms in a Ghanaian population' BMC MEDICAL GENETICS vol. 10, no. 124, 02 December 2009, pages 1 - 6, XP021066767
- CHIBA, K: 'Genetic polymorphism of the CYP2C subfamily' FOLIA PHARMACOL. JPN. vol. 112, no. 1, 1998, pages 15 - 21, XP055139478
- SHUEN-IU HUNG ET AL.: 'Common risk allele in aromatic antiepileptic-drug induced Stevens-Johnson syndrome and toxic epidermal necrolysis in Han Chinese' PHARMACOGENOMICS vol. 11, no. 3, 2010, pages 349 - 356, XP008173148
- J T WILSON ET AL.: 'High incidence of a concentration-dependent skin reaction in children treated with phenytoin' BRITISH MEDICAL JOURNAL vol. 1, 1978, pages 1583 - 1586, XP055139480
- WEN-HUNG CHUNG ET AL: "Genetic predisposition of life-threatening antiepileptic-induced skin reactions", EXPERT OPINION ON DRUG SAFETY, vol. 9, no. 1, 15 December 2009 (2009-12-15), pages 15-21, XP055270441, GB ISSN: 1474-0338, DOI: 10.1517/14740330903427969

**Description**

FIELD OF THE INVENTION

The present invention is related to a method for predicting the risk of a patient for adverse drug reactions, and more particularly to a risk assessment for developing adverse drug reactions in response to phenytoin.

BACKGROUND OF THE INVENTION

Adverse drug reactions (abbreviated ADRs) are expressions that describe harm associated with the use of given medication at a normal dosage. Drug hypersensitivity is a common adverse event during medical treatments and contributes to about 20% of reported ADRs. These hypersensitivity reactions may present from mild skin rash (MPE) to life-threatening drug reaction with eosinophilia and systemic symptoms (DRESS), Stevens-Johnson syndrome (SJS), or toxic epidermal necrolysis (TEN).

Many aromatic antiepileptic drugs (AEDs), such as phenytoin, carbamazepine or lamotrigine, are frequently associated with hypersensitive reactions. In particular, phenytoin is a first-line AED, however, more than 19% of patients received phenytoin developed hypersensitivity reactions. Previous studies showed that the deficiency of microsomal epoxide hydroxylase and human leukocyte antigen (HLA) subtypes may associate with AED hypersensitivity. However, the relationship between drug metabolism/genetic susceptibility and phenytoin-induced hypersensitivity reactions is still unclear.

Therefore, there remains a need for a new and improved method for predicting the risk of phenytoin-induced hypersensitivity, wherein the risk can be assessed by evaluating the factors including genetic polymorphisms of CYP2C9, phenytoin plasma concentration and HLA genotypes.

SUMMARY OF THE INVENTION

The article by S.-I. Hung et al. with the title "Common risk allele in aromatic antiepileptic-drug induced Stevens-Johnson syndrome and toxic epidermal necrolysis in Han Chinese", PHARMACOGENOMICS, vol. 11, no. 3, 1 January 2010, pages 349-356, ISSN: 1462-2416, and corresponding patent application WO2005/047544A1 both disclose HLA alleles that were identified in a population-based case-control study as common risk alleles for Stevens-Johnson syndrome and toxic epidermal necrolysis.

The article by William Kudzi et al. with the title "Characterisation of CYP2C8, CYP2C9 and CYP2C19 polymorphisms in a Ghanaian population,", BMC MEDICAL GENETICS, vol. 10, no. 1, 2 December 2009, page 124, ISSN: 1471-2350 describes the use of "RFLP" and "direct sequencing" methods for determining the frequencies of 9 SNPs on CYP2C genes from 204 healthy subjects in a population of Ghanaian. According to the article, "healthy subjects" were enrolled and there were no patients with phenytoin-induced Stevens-Johnson syndrome, phenytoin- induced toxic epidermal necrolyis or phenytoin-induced drug reactions with eosinophilia and systemic symptoms (DRESS). According to the article, 3 SNPs (CYP2C8*2, CYP2C9*11 and CYP2C19*2) were found showing polymorphism in the general population, and the remaining 6 SNPs showed non-polymorphic (allele frequency: 0%) in the general population. The 3 polymorphic SNPs are present in the general population, and do not have association to phenytoin hypersensitivity. Further, the article indicates that genetic polymorphisms of the CYP2C subfamily of enzymes are thought to influence both efficacy of drugs and the likelihood of ADRs of some drugs and discloses that phenytoin-induced neurological toxicity was reported in some patients carrying alleles of CYP2C9.

The article by Robert T. Kinobe et al. with the title "P450 2C18 Catalyzes the Metabolic Bioactivation of Phenytoin", CHEMICAL RESEARCH IN TOXICOLOGY, vol. 18, no. 12, 1 December 2005, pages 1868-1875, ISSN: 0893-228X, DOI: 10.1021/tx0501810 discloses that CYP2C18 may be important for the extrahepatic tissue-specific bioactivation of PHT in vivo and mentions that the safe clinical use of phenytoin is comprised by a drug hypersensitivity reaction (characterized by rash, lymphadenopathy, fever and hepatitis). The mechanism to cause rash, lymphadenopathy, fever and hepatitis is different from the mechanism to cause SJS, TEN and DRESS, which are severe cutaneous adverse reactions.

The article by López Marisol et al. with the title "Pharmacogenetics of the antiepileptic drugs phenytoin and lamotrigine", DRUG METABOLISM AND DRUG INTERACTIONS, vol. 26, no. 1, May 2011, pages 5-12, ISSN: 0792-5077 discloses the CYP2C9*2, CYP2C9*3 and CYP2C19*2 alleles affect the PHT plasma concentration and toxicity. Several case studies reported that PHT induced central nervous system toxicities were observed when the patients were carries of defective CYP2C9 and/or CYP2C19 alleles.

In addition, the article also points out that concerning the development of cutaneous adverse drug reactions during PHT therapy, it is not yet clear whether there is an association with CYP2C9/ CYP2C19 polymorphisms.

The article by Junji Saruwatari et al. with the title "Update on the genetic polymorphisms of drug-metabolizing enzymes in antiepileptic drug therapy", PHARMACEUTICALS, vol. 3, no. 8, 20 August 2010, pages 2709-2732, ISSN:

1424-8247, DOI: 10.3390/PH3082709 discloses that the CYP2C9*2, CYP2C9*3 and CYP2C19*2 alleles have all been shown to affect the phenytoin plasma concentration and toxicity and phenytoin-induced central nervous system toxicities were observed when the patients were carriers of defective CYP2C9 and/or CYP2C19 allele(s).

The article by J. A. Goldstein with the title "Clinical relevance of genetic polymorphisms in the human CYP2C subfamily", BRITISH JOURNAL OF CLINICAL PHARMACOLOGY, vol. 52, no. 4, 1 October 2001, pages 349-355, ISSN: 0306-5251, DOI: 10.1046/J. 0306-5251.2001.01499.X discloses that genetic polymorphisms of CYP2C9 and CYP2C19 have been shown to have clinical consequences resulting in toxicity of some drugs in the affected individual, and may alter efficacy of other drugs. Polymorphisms in CYP2C9 have the potential to affect the toxicity of drugs with somewhat lower therapeutic indices such as warfarin, phenytoin, and certain antidiabetic drugs. Clinical problems with toxicity and dosage adjustment of both warfarin and phenytoin have been found in *CYP2C9* PMs (poor metabolizers).

The following articles review the published work with particular emphasis on pharmacogenetic alterations that may affect efficiency, tolerability, and safety of antiepileptic drugs (AEDs), including variation in genes encoding drug metabolizing enzymes (CYP2C9, CYP2C19): The article by Löscher Wolfgang et al. with the title "The clinical impact of pharmacogenetics on the treatment of epilepsy", EPILEPSIA, vol. 50, no. 1, 1 January 2009, pages 1-23, ISSN: 0013-9580, DOI: 10.1111/j. 1528-1167.2008.01716.x, and the article by Ulrich Klotz with the title "The role of pharmacogenetics in the metabolism of antiepileptic drugs - Pharmacokinetic and therapeutic implications!", CLINICAL PHARMACOKINETICS, vol. 46, no. 4, 2007, pages 271-279, ISSN: 0312-5963. Both of these articles indicate that the metabolic ratio (HPPH/PHT) was significantly lower in all mutant genotypes if compared with the wild-type *CYP2C9/ CYP2C19;* likewise, plasma levels of PHT were higher in the subjects with allele variants and leading toxicity.

In addition, Löscher et al. also points out that concerning the development of cutaneous adverse drug reactions during PHT therapy, it is not yet clear whether there is an association with CYP2C9/ CYP2C19 polymorphisms.

The article by Brandolese et al. with the title "Severe phenytoin intoxication in a subject homozygous for CYP2C9*3", CLINICAL PHARMACOLOGY AND THERAPEUTICS, vol. 70, no. 4, 1 October 2001, pages 391-394, ISSN: 0009-9236 is a case report of a patient who carries CYP2C9*3 allele and CYP2C19*2 allele and manifested neurologic signs compatible with phenytoin intoxication. The symptoms described are all central nervous system toxicities which belonged to "type A" reactions (drug toxicity), but not "type B" reactions (drug allergy) (especially SCARs).

US 2006/269935 A1 discloses adverse drug reactions as neurological side effects due to drug toxicity reactions.

From W.-H. Chung et al., Genetic predisposition of life-threatening antiepileptic-induced skin reaction, Expert Opin. Drug Saf. (2010) 9(1):15-21, it is known that adverse drug reactions can be broadly divided into two types, type A and type B. Type A reactions are considered as a magnification of a drug's pharmacologic effect. The reactions are an augmentation of the normal pharmacological actions of the drug and caused by drug toxicity. This type of condition is predictable from the known pharmacology of a drug and typically dose dependent. Type B reactions are drug hypersensitivity reactions (drug allergy) which are very rare, unpredictable, and do not involve the pharmacological effects of a drug. The prior art cited above refers to Type A reactions, while the present invention is directed to type B reactions.

There remains a need for a new and improved method for predicting the risk of phenytoin-induced hypersensitivity, wherein the risk can be assessed by evaluating the factors including genetic polymorphisms of CYP2C9, phenytoin plasma concentration and HLA genotypes.

SUMMARY OF THE INVENTION

The present invention is defined in the claims. It relates to a method for assessing the risk of a patient for developing phenytoin-induced adverse drug reactions, particularly for SJS, TEN, and DRESS that are severe cutaneous adverse reactions by using genome wide association study (Affymetrix 6.0) of 58 phenytoin-induced severe cutaneous adverse drug reactions (SCARs), including SJS, TEN, DRESS and 198 controls. In one embodiment, the method for accessing the risk of a patient for developing phenytoin-induced adverse drug reactions comprises the step of detecting the presence of SNPs (Single Nucleotide Polymorphism) on chromosome 10 in the CYP2Cs region associated with phenytoin-induced hypersensitivity, such as rs17110192, rs7896133 on CYP2C18; rs1057910 (CYP2C9*3), rs17110321, rs9332093, rs9332245 on CYP2C9; rs3758581, rs2860905, rs4086116 on CYP2C19 and rs7899038, rs1592037, rs1934952, rs11572139, rs6583967 on CYP2C8.

In another embodiment, the method for assessing the risk of a patient for developing phenytoin-induced adverse drug reactions comprises the step of combining rs1057910 (CYP2C9*3), rs3758581, rs17110192, rs9332245 or rs1592037 (especially for rs17110192, rs1057910 and rs3758581) belonging to a haplotype that can increase the statistical significance of association with phenytoin-induced SJS/TEN/DRESS comparing to phenytoin tolerant controls. There was no phenytoin tolerant individuals carried the combination of rs1057910, rs3758581, rs17110192, rs9332245 or rs1592037 (especially for rs1057910 and rs3758581, 0/95), however, near one third of phenytoin hypersensitivity that received phenytoin more than 3 months without any adverse reaction. The result showed there are up to 30.2% of phenytoin-SJS/TEN and 37.5% of phenytoin-DRESS carried *CYP2C9*3* genotype. In comparison, 14.7% of phenytoin-MPE and only 2.5% of tolerant controls carried *CYP2C9*3.* The present invention also indicates that genetic variants in CYP2C9 as well as in CYP2C19, CYP2C8 and CYP2C18 are related to poor metabolic activity for phenytoin and increase drug accumulation in plasma that leads to hypersensitivity reactions. In addition to the genetic link in CYP2Cs, *HLA-A\*0207, HLA-A\*2402, HLA-B\*1301, HLA-B\*1502, HLA-B\*4001, HLA-B\*4609, HLA-B\*5101, HLA-DRB1\*1001 or HLA-DRB1\*1502* also showed significant association with phenytoin-induced hypersensitivity. Thus, these data revealed that the genetic polymorphisms of CYP2C9/2C19/2C8/2C18 or its defective alleles, the phenytoin plasma levels and specific HLA genotypes all contribute to phenytoin induced severe cutaneous adverse drug reactions.

BRIEF DESCRIPTION OF THE DRAWINGS

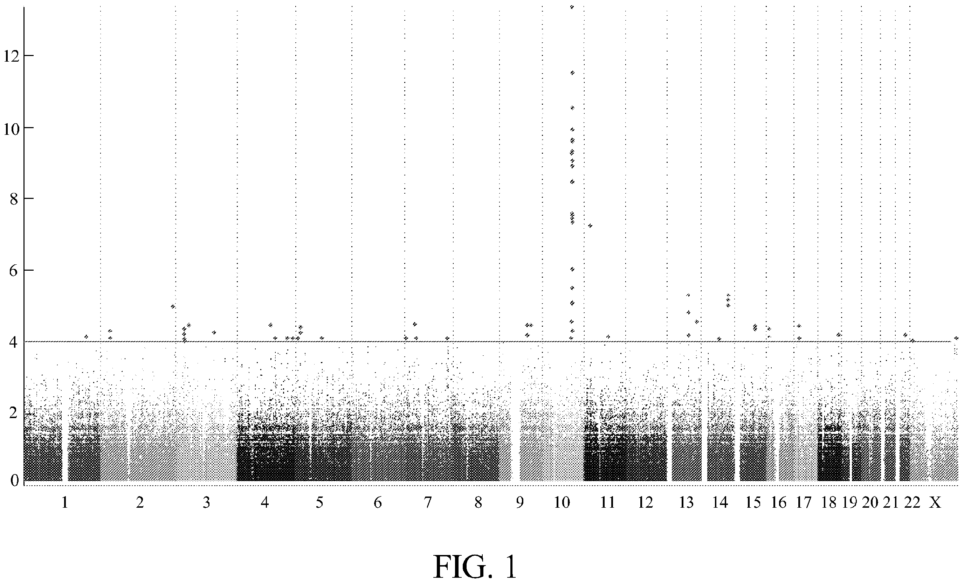

FIG. 1 illustrates the genome-wide association study of phenytoin-induced SCARs, where each dot represents a SNP. The x-axis represents the position of the SNP on chromosomes, while the y-axis represents the $-\log_{10}$ P value of the SNP in the case-control association study. 58 phenytoin-induced SJS/TEN/DRESS and 198 population controls are included in the study. SNPs with P values $<10^{-6}$ is highlighted in red. The strong signal in chromosome 10 lies in the CYP2C region.

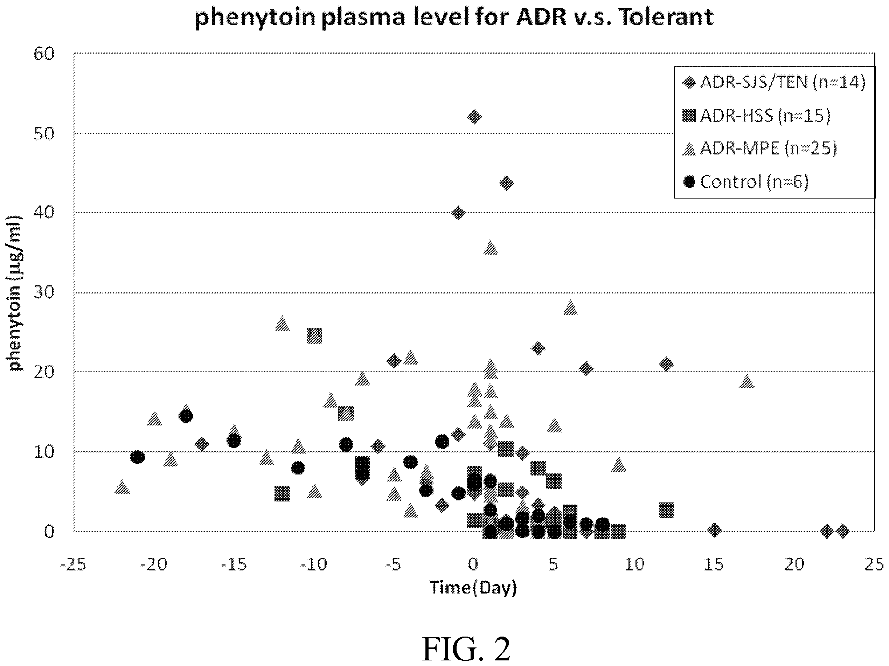

FIG. 2 shows phenytoin plasma level of phenytoin-ADRs and tolerant controls, where the normal therapeutic levels of phenytoin are usually between 10-20 $\mu$g/ml and the phenytoin plasma levels of tolerant controls were between 5-15 $\mu$g/ml. However, the phenytoin plasma levels of patients with phenytoin-SJS/TEN were 40-50 $\mu$g/ml that were higher than that of tolerant controls.

DETAILED DESCRIPTION OF THE INVENTION

FIG. 1 illustrates the genome-wide association study of phenytoin-induced SCARs, where each dot represents a SNP. The x-axis represents the position of the SNP on chromosomes, while the y-axis represents the $-\log_{10}$ P value of the SNP in the case-control association study. 58 phenytoin-induced SJS/TEN/DRESS and 198 population controls are included in the study. SNPs with P values $< 10^{-6}$ is highlighted in red. The strong signal in chromosome 10 lies in the CYP2C region.

FIG. 2 shows phenytoin plasma level of phenytoin-ADRs and tolerant controls, where the normal therapeutic levels of phenytoin are usually between 10-20 $\mu$g/ml and the phenytoin plasma levels of tolerant controls were between 5-15 $\mu$g/ml. However, the phenytoin plasma levels of patients with phenytoin-SJS/TEN were 40-50 $\mu$g/ml that were higher than that of tolerant controls.

DETAILED DESCRIPTION OF THE INVENTION

The detailed description set forth below is intended as a description of the presently exemplary device provided in accordance with aspects of the present invention and is not intended to represent the only forms in which the present invention may be prepared or utilized. It is to be understood, rather, that the same or equivalent functions and components may be accomplished by different embodiments that are also intended to be encompassed within the scope of the invention.

Unless defined otherwise, all technical and scientific terms used herein have the same meaning as commonly understood to one of ordinary skill in the art to which this invention belongs. Although any methods, devices and materials similar or equivalent to those described can be used in the practice or testing of the invention, the exemplary methods, devices and materials are now described.

All publications are mentioned for the purpose of describing and disclosing, for example, the designs and methodologies that are described in the publications that might be used in connection with the presently described invention. The publications listed or discussed above, below and throughout the text are provided solely for their disclosure prior to the filing date of the present application. Nothing herein is to be construed as an admission that the inventors are not entitled to antedate such disclosure by virtue of prior invention.

The diagnosis of Stevens-Johnson syndrome (SJS) and toxic epidermal necrolysis (TEN) were based on the clinical morphology defined by consensus criteria (Bastuji-Garin S, Rzany B et al, 1993). SJS is defined as skin detachment of <10% of body-surface area, overlap SJS_TEN as skin detachment of 10-29% and TEN as >/= 30%. The criteria for DRESS were cutaneous rash (e.g. diffuse macuopapular, exfoliative dermatitis) with symptoms of eosinophilia, atypical circulating lymphocytes, acute hepatocellular injury, or worsening renal function (Kardaun SH, Sidoroff A et al, 2007). Patients who fulfilled the diagnostic criteria of SJS, TEN, or DRESS induced by phenytoin were identified in Chang Gung Memorial Hospital Health System and were enrolled for this study.

It was discovered that specific CYP2C genetic variants, including CYP2C9, CYP2C19, CYP2C8 and CYP2C18 were related to low metabolic activity of phenytoin and was associated with phenytoin induced hypersensitivity reactions.

In addition to CYP2C variants, HLA-A*0207, HLA-A*2402, HLA-B*1301, HLA-B*1502, HLA-B*4001, HLA-B*4609, HLA-B*5101, HLA-DRB1*1001 or HLA-DRB1*1502 were also discovered to show significant association with phenytoin-induced hypersensitivity or cutaneous adverse drug reactions.

adverse drug reactions induced by phenytoin. 152 patients were enrolled for the study of phenytoin-induced hypersensitivity reactions, including 53 cases of phenytoin-SJS/TEN, 24 cases of phenytoin-DRESS and 75 cases of phenytoin-MPE, as well as 118 tolerant controls that received phenytoin more than 3 months without any adverse reaction. The results showed that CYP2C9*3 variant was present in 16 of 53 (30.2%) phenytoin-induced SJS/TEN patients, 9 of 24 (37.5%) phenytoin-induced DRESS patients and 11 of 75 (14.7%) phenytoin-induced MPE. In comparison, the variant was only found in 2.5% (3/118) of the phenytoin-tolerant group (see Table 2). Statistical analysis of phenytoin hypersensitivity cases and tolerant controls showed that CYP2C9*3 was most significantly associated with phenytoin induced SJS/TEN (SJS/TEN vs. tolerant controls : $P$=3.3x10$^{-7}$, OR=17.5 (4.8-63.7) or DRESS (DRESS vs. tolerant controls : P =6.1x10$^{-5}$, OR=19.2 (4.4-82.7)), but only weakly associated with phenytoin-MPE (MPE vs. tolerant control : P=0.004, OR=6.2 (1.6-23.3)), suggesting that the presence of this CYP2C9*3 variant can be used in the identification of high-risk patients for phenytoin-induced ADRs, particularly phenytoin-induced SJS/TEN or DRESS.

In another embodiment, the SNPs on CYP2C causing the amino acid to change can also be used for assessing the risk of phenytoin-induced adverse drug reactions. A further study has been conducted for some SNPs on CYP2C genes which can make amino acid changed, such as 371G > A (rs12414460, Arg124Gln), 895A > G, (rs72558192, Thr299Ala, known as *CYP2C9*16*), 1362G > C (Gln454His, known as *CYP2C9*19*) on CYP2C9; 991A> G (rs3758581, Ile331Val), 395G > A (rs72558184, Arg132Gln, known as *CYP2C19*6*), 636G > A (rs4986893, Trp212end, known as *CYP2C19*3*), 681G > A, (rs4244285, causing splicing site mutation, known as *CYP2C19*2*), 781C > T (Arg261Trp) on CYP2C19 and 204T > A (rs41291550 ' Tyr68end), 370C > T (Arg124Trp), 576G > C (Gln192His), 1154C > T (rs2281891 ' Thr385Met) on CYP2C18. These changes of CYP2C enzyme can affect both its activity and its substrate specificity. For example, rs3758581, a missense change in CYP2C19 exon7 was significantly associated with phenytoin-SJS/TEN/DRESS (SJS/TEN/DRESS vs. Tolerant : P=0.0003, OR=7.28 (2.3564-22.4912)) (see Table 1).

**Table 1: List of SNPs of CYP2C loci in chromosome 10 significantly associated with phenytoin-SJS/TEN/DRESS**

| | | | % A, B | | |
|---|---|---|---|---|---|
| RS# | Annotation | Chr | Fisher_P | OR | |
| rs17110192 | CYP2C18 | 10 | 4.03E-14 | 13.33333 | |
| rs7896133 | CYP2C18 | 10 | 1.14E-06 | 0.115132 | |
| rs17110321 | CYP2C9 | 10 | 4.03E-14 | 0.075 | |
| rs9332093 | CYP2C9 | 10 | 1.19E-13 | 13.00438 | |
| rs9332245 | CYP2C9 | 10 | 4.37E-13 | 12.5 | |
| rs2860905 | CYP2C19 | 10 | 1.14E-06 | 0.115132 | |
| rs4086116 | CYP2C19 | 10 | 3.49E-05 | 7.6 | |
| rs3758581 | CYP2C19 | 10 | 3.00E-04 | 7.28 | |
| rs7899038 | CYP2C8 | 10 | 1.87E-07 | 0.078083 | |
| rs1592037 | CYP2C8 | 10 | 4.92E-13 | 11.67059 | |
| rs1934952 | CYP2C8 | 10 | 7.39E-10 | 6.441558 | |
| rs11572139 | CYP2C8 | 10 | 6.38E-06 | 15.32967 | |
| rs6583967 | CYP2C8 | 10 | 4.92E-13 | 11.67059 | |
| rs11188183 | chromosome 10 open reading frame 129 | 10 | 3.06E-12 | 0.083365 | |
| rs7921561 | chromosome 10 open reading frame 129 | 10 | 5.52E-13 | 11.6098 | |
| rs644437 | chromosome 10 open reading frame 129 | 10 | 4.92E-13 | 11.67059 | |

(continued)

| RS# | Annotation | Chr | % A, B | |
| | | | Fisher_P | OR |
|---|---|---|---|---|
| rs12769577 | chromosome 10 open reading frame 129 | 10 | 4.92E-13 | 11.67059 |
| rs10882551 | chromosome 10 open reading frame 129 | 10 | 4.96E-14 | 14.61539 |
| rs585381 | chromosome 10 open reading frame 129 | 10 | 4.31E-12 | 0.096042 |
| rs648638 | chromosome 10 open reading frame 129 | 10 | 4.92E-13 | 11.67059 |
| rs664093 | chromosome 10 open reading frame 129 | 10 | 4.92E-13 | 0.085685 |
| rs12262878 | chromosome 10 open reading frame 129 | 10 | 1.67E-13 | 12.76471 |
| rs17524438 | chromosome 10 open reading frame 129 | 10 | 1.26E-12 | 0.0875 |

**Table 2: CYP2C9*3 frequency in 152 patients with phenytoin-induced cutaneous ADRs and 118 tolerant controls**.

| CYP2C9 Genotypes | CYP2C9*1 | CYP2C9*3 § | Total |
|---|---|---|---|
| SJS/TEN | 37 (69.8%) | 16 (30.2%) | 53 |
| DRESS | 15 (62.5%) | 9 (37.5%) | 24 |
| MPE | 64 (85.3%) | 11 (14.7%) | 75 |
| Tolerance | 115 (97.5%) | 3(2.5%) | 118 |

§: SJS/TEN vs. Tolerant :
$P$=3.3x10$^{-7}$,
OR=17.5 (4.8-63.7); DRESS vs. Tolerant :
$P$ =6.1x10$^{-5}$, OR=19.2 (4.4-82.7); MPE vs. Tolerant :
$P$=0.004, OR=6.2 (1.6-23.3)

In still another embodiment in the present invention, different SNPs can be combined for assessing the risk of phenytoin-induced adverse drug reactions. It is found that a combination of rs1057910 (CYP2C9*3), rs3758581, rs17110192, rs9332245 or rs1592037 belonging to a haplotype can increase the statistical significance of association with phenytoin-induced SJS/TEN/DRESS comparing to phenytoin tolerant controls. Especially when patients carry both rs1057910 and rs3758581 or rs17110192 have more significant association with phenytoin-induced SJS/TEN/DRESS (see Table 3).

**Table 3: Statistical analysis of major SNPs associated with phenytoin-induced SJS/TEN/DRESS**

| | | PHT-Case (n=126) | | Control (n=95) | | Fisher's test | Chi-square | Odds ratio | 95% CI |
|---|---|---|---|---|---|---|---|---|---|
| 6 SNPs | rs1592037, rs9332245, rs1057910, rs3758581, rs4986893, rs17110192 | 2 | 124 | 0 | 95 | 0.50761004 | 0.21731708 | 3.0645 | 0.137 - 68.746 |

(continued)

|  | | PHT-Case (n=126) | | Control (n=95) | | Fisher's test | Chi-square | Odds ratio | 95% CI |
|---|---|---|---|---|---|---|---|---|---|
| 5 SNPs | rs1592037, rs9332245, rs1057910, rs3758581, rs17110192 | 29 | 97 | 0 | 95 | 1.93E-08 | 5.30E-07 | 56.8041 | 3.4201 - 943.446 |
| 4 SNPs | rs1592037, rs1057910, rs3758581, rs17110192 | 29 | 97 | 0 | 95 | 1.93E-08 | 5.30E-07 | 56.8041 | 3.420 - 943.446 |
| | rs1592037, rs9332245, rs1057910, rs17110192 | 31 | 95 | 1 | 94 | 9.93E-08 | 8.40E-07 | 30.6737 | 4.103 - 229.311 |
| | rs1592037, rs9332245, rs3758581, rs17110192 | 29 | 97 | 2 | 93 | 2.7454E-06 | 0.00000936 | 13.9021 | 3.226 - 59.915 |
| 3 SNPs | rs1057910, rs3758581, (rs17110192 / rs9332245) | 30 | 96 | 0 | 95 | 9.50E-09 | 3.10E-07 | 59.375 | 3.578 - 985.443 |
| | rs9332245, rs17110192, (rs3758581 / rs1592037) | 29 | 97 | 3 | 92 | 1.3629E-05 | 0.0000328 | 9.1684 | 2.700 - 31.131 |
| | rs1592037, rs9332245, rs3758581 | 29 | 97 | 2 | 93 | 2.7454E-06 | 0.00000936 | 13.9021 | 3.226 - 59.915 |
| | rs1592037 / rs9332245 / rs3758581 / rs17110192 | 30 | 96 | 3 | 92 | 7.3945E-06 | 0.00002005 | 9.5833 | 2.827 - 32.487 |
| 2 SNPs | rs1057910, rs17110192 | 31 | 95 | 1 | 94 | 9.93E-08 | 8.40E-07 | 30.6737 | 4.103 - 229.311 |
| | rs1057910, rs3758581 | 30 | 96 | 0 | 95 | 9.50E-09 | 3.10E-07 | 59.375 | 3.5778 - 985.443 |
| | rs1057910, (rs1592037 / rs9332245 / rs3758581) | 30 | 96 | 1 | 94 | 2.05E-07 | 1.42E-06 | 29.375 | 3.926 - 219.805 |

In a further aspect, the present disclosure provides a method of assessing the risk of phenytoin-induced adverse drug reactions comprising the step of identifying the presence of HLA genotypes, including HLA-A, HLA-B and HLA-DRB1. PCR-SSO is used to identify the HLA-A, HLA-B and HLA-DRB1 genotype of phenytoin-ADRs patients and tolerant controls. The results indicated that *HLA-A\*0207, HLA-A\*2402, HLA-B\*1301, HLA-B\*1502, HLA-B\*4001, HLA-B\*4609, HLA-B\*5101, HLA-DRB1\*1001 or HLA-DRB1\*1502* were significantly associated with phenytoin-induced ADRs (SJS/TEN, DRESS, or MPE) (see Table 4 to 6). The data shows that HLA-B\*1301 allele significantly increased frequencies among patients with phenytoin-ADRs compared to the tolerant controls. There were 15 SJS/TEN patients (28.3%) and 12 DRESS patients (46.2%) carried HLA-B*1301, while only 14 tolerant patients (11.9%) carried this genotype (SJS/TEN vs. tolerant controls: $P$=0.001, OR=3.8 (1.7-8.5); DRESS vs. tolerant controls: P =$2x10^{-4}$, OR=6.4 (2.5-16.5)). However, this association was not significant in MPE (MPE vs. Tolerant controls: P=0.296, OR=1.7 (0.7-3.7)). HLA-B*5101 allele was also associated with phenytoin-induced ADRs. There were 13.2% SJS/TEN patients, 19.2% DRESS patients and 15.6% MPE patients carried HLA-B*5101, while only 5 tolerant controls (4.2%) carried this genotype (SJS/TEN vs. tolerant controls: P=0.05, OR=3.4; DRESS vs. tolerant controls: P=0.018, OR=5.4; MPE vs. tolerant controls: P=0.009, OR=4.2).

Table 4. Associations between *HLA-A* alleles and phenytoin-induced cutaneous ADR

| *HLA-A* allele | Tolerant (n=117) | SJS/TEN (n=53) | p-value | Odds ratio (95% CI) | DRESS (n=22) | p-value | Odds ratio (95% CI) | MPE (n=75) | p-value | Odds ratio (95% CI) |
|---|---|---|---|---|---|---|---|---|---|---|
| 0207 | 7 (6.0%) | 7 (13.2%) | 0.135 | 2.4 (0.8-7.2) | 5 (22.7%) | 0.024 | 4.6 (1.3-16.2) | 9 (12.0%) | 0.182 | 2.1 (0.8-6.0) |
| 2402 | 38 (32.5) | 8 (15.1%) | 0.025 | 0.4 (0.2-0.9) | 4 (18.2%) | 0.214 | 0.5 (0.1-1.5) | 20 (26.7%) | 0.424 | 0.8 (0.4-1.4) |

**Table 5. Associations between *HLA-B* alleles and phenytoin-induced cutaneous ADR**

| *HLA-B* allele | Tolerant (n=118) | SJS/TEN (n=53) | p-value | Odds ratio (95% CI) | DRESS (n=26) | p-value | Odds ratio (95% CI) | MPE (n=77) | p-value | Odds ratio (95% CI) |
|---|---|---|---|---|---|---|---|---|---|---|
| 1301 | 14 (11.9%) | 15 (28.3%) | 0.001 | 3.8 (1.7-8.5) | 12 (46.2%) | $2 \times 10^{-4}$ | 6.4 (2.5-16.5) | 14 (18.2%) | 0.296 | 1.7 (0.7-3.7) |
| 1502 | 9 (7.6%) | 12 (22.6%) | 0.010 | 3.5 (1.4-9.0) | 0 (0.0%) | 0.213 | 0.2 (0.0-4.2) | 8 (10.4%) | 0.605 | 1.4 (0.5-3.8) |
| 5101 | 5 (4.2%) | 7 (13.2%) | 0.050 | 3.4 (1.0-11.4) | 5 (19.2%) | 0.018 | 5.4 (1.4-20.2) | 12 (15.6%) | 0.009 | 4.2 (1.4-12.4) |
| 4609 | 0 (0%) | 0 (0%) | 1.000 | - | 2 (7.7%) | 0.032 | 19.7 (0.9-449.8) | 0 (0%) | 1.000 | - |
| 4001 | 52 (44.1%) | 20 (37.7%) | 0.504 | 0.8 (0.4-1.5) | 9 (34.6%) | 0.393 | 0.7 (0.3-1.6) | 22 (28.6%) | 0.035 | 0.5 (0.3-1.9) |

Our previous study has showed that HLA-B*1502 allele was strongly associated with carbamazepine-induced SJS/TEN (Chung WH et al, 2004). In still a further aspect of the present disclosure, HLA-B*1502 allele is used for assessing the risk of phenytoin-induced adverse drug reactions, and the results showed that HLA-B*1502 was associated with phenytoin-induced ADRs. There were 12 SJS/TEN patients (22.6%) carried HLA-B*1502, while only 9 tolerant controls (7.6%) carried this genotype. HLA-B*1502 allele significantly increased frequencies among the phenytoin-induced SJS/TEN patients compared to the tolerant controls (SJS/TEN vs. tolerant: P=0.01, OR=3.5 (1.4-9.0)). However, this association was not seen in phenytoin-induced DRESS and MPE (P> 0.05).

In addition, in the present disclosure, we also found that HLA-B*4609 allele significantly increased frequencies among the phenytoin-induced DRESS patients compared to the tolerant controls (DRESS vs. tolerant controls: P=0.032, OR=19.7 (0.9-449.8)) and HLA-A*0207 allele was also associated with phenytoin-induced DRESS (DRESS vs. tolerant controls: P=0.024, OR=4.6 (1.3-16.2)). Moreover, HLA-DRB1*1001 was found to be associated with phenytoin-induced MPE (MPE vs. tolerant controls: P=0.02, OR=13.2); HLA-DRB1*1502 was found to be associated with phenytoin-induced SJS/TEN (SJS/TEN vs. tolerant controls: P=0.029, OR=14). In contrast, HLA-A*2402 and HLA-B*4001 allele were showed a protective effect in phenytoin-induced ADRs (decreased allele frequencies in phenytoin-induced ADRs, increased in tolerant controls).

**Table 6, Associations between *HLA*-*DRB1* alleles and phenytoin-induced cutaneous ADRs**

| *HLA -DRB1* allele | Tolerant (n=117) | SJS/TEN (n=53) | p-value | Odds ratio (95% CI) | DRESS (n=22) | p-value | Odds ratio (95% CI) | MPE (n=75) | p-value | Odds ratio (95% CI) |
|---|---|---|---|---|---|---|---|---|---|---|
| 1001 | 0(0.0%) | 0 (0.0%) | 1.000 | - | 0 (0.0%) | 1.000 | - | 4 (5.3%) | 0.022 | 13.2 (0.7-253.1) |
| 1502 | 0 (0.0%) | 3 (5.7%) | 0.029 | 14.0 (0.7-285.5) | 0 (0.0%) | 1.000 | - | 0 (0.0%) | 1.000 | |

The study also indicates that genetic variants in CYP2C9 as well as in CYP2C19, CYP2C8, CYP2C18 are related to poor metabolic activity for phenytoin and increase its drug accumulation that leads to the risk of phenytoin hypersensitivity. In some embodiments, the method for assessing the risk of phenytoin-induced adverse drug reactions comprises the step of detecting phenytoin concentration in plasma. Since phenytoin is metabolized by liver enzymes, polymorphisms of cytochrome P450 enzymes can affect phenytoin plasma concentrations. The non-linear pharmacokinetics of phenytoin and narrow therapeutic window suggesting phenytoin concentrations in plasma may be related to its efficacy and toxicity.

In this study, the phenytoin concentrations during ADR onset were determined from patients' plasma by HPLC analysis and estimated by non-linear pharmacokinetics formula:

$$t = \frac{K_{\mathrm{m}} \times \ln\left(\frac{C_1}{C_2}\right) + (C_1 - C_2)}{V_{\max}} \times V_{\mathrm{d}}$$

Km = 4 mg/L (substrate concentration at which the rate of metabolism is one half of Vmax)
**Vmax = 7 mg/kg/day** (maximum metabolic rate)
**Vd = 0.65 L/kg** (volume of distribution)
t: the time (days) between any two phenytoin plasma concentration

Therapeutic levels of phenytoin are usually between 10-20 $\mu$g/ml. After taking drug, phenytoin plasma levels of tolerant control patients were between 5-15 $\mu$g/ml. However, in SJS/TEN patients, the phenytoin plasma levels were up to 40-50 $\mu$g/ml that were higher than tolerant control patients (SJS/TEN vs. tolerant control: P=0.006; MPE vs. tolerant controls: P=0.004) (see FIG. 2). We also compared the phenytoin plasma levels of patients who carried CYP2Cs variants. The result showed that phenytoin-ADRs patients who carried CYP2C9*3 were with higher phenytoin plasma levels. After analyzing the phenytoin level of 23 phenytoin-ADRs patients, there were 4 patients whose phenytoin levels were lower than 10$\mu$g/ml, 9 patients' phenytoin levels were between 10-20$\mu$g/ml and 10 patients' phenytoin levels were higher than 20$\mu$g/ml. Among these 23 patients, the phenytoin concentrations of 5 patients who carry CYP2C9*3 variant were all higher than 20$\mu$g/ml. The invention proves CYP2Cs variants, especially CYP2C9*3, display poor metabolic activity of phenytoin and can result in hypersensitivity reactions. Defective metabolism of phenytoin may increase the risk of drug accumulation, and leads to its risk to induce severe hypersensitivity reactions, such as SJS/TEN and DRESS.

The CYP2Cs variants and HLA genotypes can be detected by using any method known in the art. Preferably, genomic DNA is hybridized to a probe that is specific for the variant of interest. The probe may be labeled for direct detection, or contacted by a second, detectable molecule that specifically binds to the probe. Alternatively, cDNA, RNA, or protein product of the variant can be detected.

Having described the invention by the description and illustrations above, it should be understood that these are exemplary of the invention and are not to be considered as limiting. Accordingly, the invention is not to be considered as limited by the foregoing description, but includes any equivalents.

<u>REFERENCES</u>

1. Ingelman-Sundberg, M. Pharmacogenomic biomarkers for prediction of severe adverse drug reactions. N Engl J Med 358, 637-639 (2008).
2. Bohan, K.H., et al. Anticonvulsant hypersensitivity syndrome: implications for pharmaceutical care. Pharmacotherapy. 27(10), 1425-1439 (2007).
3. Odani, A., et al. Genetic polymorphism of the CYP2C subfamily and its effect on the pharmacokinetics of phenytoin in Japanese patients with epilepsy. Clin Pharmacol Ther 62, 287-292 (1997).
4. Shintani, et al. Genetic polymorphisms and functional characterization of the 5'-flanking region of the human CYP2C9 gene: In vitro and in vivo studies. Clin Pharmacol Ther. 70(2), 175-182 (2001).
5. Lee, A.Y., Kim, M.J., Chey, W.Y., Choi, J. & Kim, B.G. Genetic polymorphism of cytochrome P450 2C9 in diphenylhydantoin-induced cutaneous adverse drug reactions. Eur J Clin Pharmacol 60, 155-159 (2004).
6. Hung, S.I., et al. Common risk allele in aromatic antiepileptic-drug induced Stevens-Johnson syndrome and toxic epidermal necrolysis in Han Chinese. Pharmacogenomics 11, 349-356 (2010).
7. Bastuji-Garin S, Rzany B, Stern RS, et al, Shear NH, Naldi L, Roujeau JC. Clinical classification of cases of toxic epidermal necrolysis, Stevens-Johnson syndrome, and erythema multiforme. Arch Dermatol 129, 92-6 (1993).

# EP 2 723 888 B1

8. Kardaun SH, Sidoroff A, Valeyrie-Allanore L, Halevy S, Davidovici BB, Mockenhaupt M, et al. Variability in the clinical pattern of cutaneous side-effects of drugs with systemic symptoms: does a DRESS syndrome really exist? Br J Dermatol 156, 609-11 (2007).

## Claims

1.  A method of assessing the risk of a patient for developing a phenytoin-induced adverse drug reaction (ADR), said adverse drug reaction being selected out of Stevens-Johnson syndrome (SJS), toxic epidermal necrolysis (TEN), and drug reaction with eosinophilia and systemic symptoms (DRESS), said method comprising the steps of:

    detecting the presence of specific SNPs (Single Nucleotide Polymorphism) in CYP2C9, CYP2C8, CYP2C18 and CYP2C19 genes on chromosome 10 from a sample of the patient; and
    associating the presence of SNPs in said CYP2C genes on chromosome 10 with an increased risk for said phenytoin-induced adverse drug reactions.

2.  The method of assessing the risk of a patient for developing a phenytoin-induced ADR of claim 1, wherein the specific SNPs are rs1057910 (CYP2C9*3), rs17110321, rs9332093, rs9332245 on CYP2C9; rs17110192 and rs7896133 on CYP2C18; rs3758581 and rs2860905 and rs4086116 on CYP2C19; and rs7899038, rs1592037, rs1934952, rs11572139 and rs6583967 on CYP2C8.

3.  The method of assessing the risk of a patient for developing a phenytoin-induced ADR of claim 1, wherein the step of associating the presence of SNPs on CYP2C9, CYP2C8, CYP2C18 and CYP2C19 on chromosome 10 with an increased risk for phenytoin-induced ADRs comprising the step of combining two or more SNPs to predict the risk for phenytoin-induced ADRs.

4.  The method of assessing the risk of a patient for developing a phenytoin-induced adverse drug reaction (ADR) of claim 1, wherein the step of detecting the presence of genetic variants on CYP2C9, CYP2C8, CYP2C18 and CYP2C19 genes on chromosome 10 includes the step of using an oligonucleotide that specifically hybridizes with the nucleic acid coding for the variant.

5.  The method of assessing the risk of a patient for developing phenytoin-induced adverse drug reaction (ADR) of claim 1, wherein the step of detecting the presence of genetic variants on CYP2C9, CYP2C8, CYP2C18 and CYP2C19 genes on chromosome 10 includes the step of using DNA prepared from the peripheral blood of the patient.

6.  The method of assessing the risk of a patient for developing a phenytoin-induced ADR of claim 1, further comprising the step of detecting a change of amino acids on CYP2C9, CYP2C8, CYP2C18 and CYP2C19 genes, wherein the change of amino acids are 1075A>C (rs1057910, Ile359Leu, known as *CYP2C9*3*), 371G > A (rs12414460, Arg124Gln), 895A > G, (rs72558192, Thr299Ala, known as *CYP2C9*16*), 1362G > C (Gln454His, known as *CYP2C9*19*) on CYP2C9; 991A> G (rs3758581, Ile331Val), 395G > A (rs72558184, Arg132Gln, known as *CYP2C19*6*), 636G > A (rs4986893, Trp212end, known as *CYP2C19*3*), 681G > A, (rs4244285, causing splicing site mutation, known as *CYP2C19*2*), 781C > T (Arg261Trp) on CYP2C19 and 204T > A (rs41291550 ' Tyr68end), 370C > T (Arg124Trp), 576G > C (Gln192His), 1154C > T (rs2281891 ' Thr385Met) on CYP2C18.

## Patentansprüche

1.  Verfahren zur Bewertung des Risikos eines Patienten für das Auftreten von phenytoin-induzierten Arzneimittelnebenwirkungen (adverse drug reaction, ADR), bei welcher die Arzneimittelnebenwirkungen ausgewählt sind aus dem Stevens-Johnson-Syndrom (SJS), toxischer epidermaler Nekrolyse (TEN) und Arzneimittelnebenwirkungen mit Eosinophilie und systemischen Symptomen (DRESS), wobei die Methode die Schritte umfasst:

    Nachweis des Vorhandenseins von spezifischen Einzelnukleotid-Polymorphismen (Single Nucleotide Polymorphisms, SNPs) in den CYP2C9-, CYP2C8-, CYP2C18- und CYP2C19-Genen auf Chromosom 10 in einer Probe von dem Patienten, und
    Assoziieren des Vorhandenseins von SNPs in den CYP2C-Genen auf Chromosom 10 mit einem erhöhten Risiko für die phenytoin-induzierten Arzneimittelnebenwirkungen.

15

**2.** Verfahren zur Bewertung des Risikos eines Patienten für das Auftreten von phenytoin-induzierten Arzneimittelnebenwirkungen (ADR) nach Anspruch 1, bei welcher die spezifischen SNPs
rs1057910 (CYP2C9*3), rs17110321, rs9332093, rs9332245 in CYP2C9,
rs17110192 und rs7896133 in CYP2C18,
rs3758581 und rs2860905 und rs4086116 in CYP2C19, und
rs7899038, rs1592037, rs1934952, rs11572139 und rs6583967 in CYP2C8 sind.

**3.** Verfahren zur Bewertung des Risikos eines Patienten für das Auftreten von phenytoin-induzierten Arzneimittelnebenwirkungen (ADR) nach Anspruch 1, bei welcher der Schritt zum Assoziieren des Vorhandenseins von SNPs in CYP2C9. CYP2C8, CYP2C18 und CYP2C19 auf Chromosom 10 mit einem erhöhten Risiko für phenytoin-induzierte Arzneimittelnebenwirkungen einen Schritt zur Kombination von zwei oder mehreren SNPs umfasst, um das Risiko für phenytoin-induzierte Arzneimittelnebenwirkungen abzuschätzen.

**4.** Verfahren zur Bewertung des Risikos eines Patienten für das Auftreten von phenytoin-induzierten Arzneimittelnebenwirkungen (ADR) nach Anspruch 1, bei welcher der Schritt zum Nachweis des Vorhandenseins von genetischen Varianten in den CYP2C9-, CYP2C8-, CYP2C18- und CYP2C19- Genen auf Chromosom 10 einen Schritt zur Verwendung eines Oligonukleotids beinhaltet, welches spezifisch mit der Nukleinsäurecodierung der Variante hybridisiert.

**5.** Verfahren zur Bewertung des Risikos eines Patienten für das Auftreten von phenytoin-induzierten Arzneimittelnebenwirkungen (ADR) nach Anspruch 1, bei welcher der Schritt zum Nachweis des Vorhandenseins von genetischen Varianten in den CYP2C9-, CYP2C8-, CYP2C18- und CYP2C19- Genen auf Chromosom 10 einen Schritt zur Verwendung von DNA beinhaltet, die aus dem peripheren Blut des Patienten gewonnen wurde.

**6.** Verfahren zur Bewertung des Risikos eines Patienten für das Auftreten von phenytoin-induzierten Arzneimittelnebenwirkungen (ADR) nach Anspruch 1, welche ferner einen Schritt zum Nachweis einer Aminosäurenänderung in den CYP2C9-, CYP2C8-, CYP2C18-und CYP2C19- Genen umfasst, wobei die Aminosäurenänderungen
1075A>C (rs1057910, Ile359Leu, bekannt als *CYP2C9*3*), 371G > A (rs12414460, Arg124Gln), 895A > G (rs72558192, Thr299Ala, bekannt als *CYP2C9*16*), 1362G > C (Gln454His, bekannt als *CYP2C9*19*) in CYP2C9,
991A> G (rs3758581, Ile331Val), 395G > A (rs72558184, Arg132Gln, bekannt als *CYP2C19*6*), 636G > A (rs4986893, Trp212end, bekannt als *CYP2C19*3*), 681G > A (rs4244285, verursacht Spleißstellenmutation, bekannt als *CYP2C19*2*), 781C > T (Arg261Trp) in CYP2C19, und
204T > A (rs41291550, Tyr68end), 370C > T (Arg124Trp), 576G > C (Gln192His), 1154C > T (rs2281891, Thr385Met) in CYP2C18 sind.

**Revendications**

**1.** Procédé d'évaluation du risque d'un patient de développer un effet indésirable d'un médicament (EIM) provoqué par la phénytoïne, ledit effet indésirable du médicament étant sélectionné parmi le syndrome de Stevens-Johnson (SJS), la nécrolyse épidermique toxique (NET) et la réaction à un médicament avec l'éosinophilie et les symptômes systémiques (DRESS) comprenant les étapes de :

détection de la présence de SNP spécifiques (polymorphisme d'un seul nucléotide) dans des gènes CYP2C9, CYP2C8, CYP2C18 et CYP2C19 sur le chromosome 10 d'un échantillon du patient et
association de la présence de SNP dans lesdits gènes CYP2C sur le chromosome 10 avec un risque accru pour lesdits effets indésirables d'un médicament provoqués par la phénytoïne.

**2.** Procédé d'évaluation du risque d'un patient de développer EIM provoqué par la phénytoïne selon la revendication 1, les SNP spécifiques étant rs1057910 (CYPC2C9*3), rs17110321, rs9332093, rs9332245 sur CYP2C9, rs17110192 et rs7896133 sur CYP2C18, rs3758581 et rs2860905 et rs4086116 sur CYP2C19 et rs7899038, rs1592037, rs1934952, rs11572139 et rs6583967 sur CYP2C8.

**3.** Procédé d'évaluation du risque d'un patient de développer EIM provoqué par la phénytoïne selon la revendication 1, l'étape d'association de la présence de SNP sur les gènes CYP2C9, CYP2C8, CYP2C18 et CYP2C19 sur le chromosome 10 avec un risque accru pour des EIM provoqués par la phénytoïne comprenant l'étape de combinaison de deux SNP ou plus pour prédire le risque d'EIM provoqués par la phénytoïne.

**4.** Procédé d'évaluation du risque d'un patient de développer EIM provoqué par la phénytoïne selon la revendication 1, l'étape de détection de la présence de variantes génétiques de gènes CYP2C9, CYP2C8, CYP2C18 et CYP2C19 sur le chromosome 10 comprenant l'étape d'utilisation d'un oligonucléotide hybridant avec l'acide nucléique codant pour la variante.

**5.** Procédé d'évaluation du risque d'un patient de développer EIM provoqué par la phénytoïne selon la revendication 1, l'étape de détection de la présence de variantes génétiques de gènes sur des gènes CYP2C9, CYP2C8, CYP2C18 et CYP2C19 sur le chromosome 10 comprenant l'étape d'utilisation d'ADN préparé à partir du sang périphérique du patient.

**6.** Procédé d'évaluation du risque d'un patient de développer EIM provoqué par la phénytoïne selon la revendication 1 comprenant de plus l'étape de détection d'acides aminés sur des gènes CYP2C9, CYP2C8, CYP2C18 et CYP2C19, le changement d'acides aminés étant 1075A>C (rs1057910), Ile359Le, connu comme *CYP2C9\*3*), 371G>A (rs12414460, Arg124Gln), 895A>G, (rs72558192, Thr299Ala, connu comme *CYP2C9\*16*), 1362G > C (Gln454His, connu comme *CYP2C9\*19*) sur CYP2C9 ; 991A>G (rs3758581, Ile331Val), 395G>A (rs72558184, Arg132Gln, connu comme *CYP2C19\*6*), 636G>A (rs4986893, Trp212end, connu comme *CYP2C19\*3*), 681G > A, (rs4244285 provoquant une mutation au site d'épissage, connu comme *CYP2C19\*2*), 781C>T (Arg261Trp) sur CYP2C19 et 204T > A (rs41291550, Tyr68end), 370C > T (Arg124Trp), 576G > C (Gln192His), 1154C>T (rs2281891, Thr385Met) sur CYP2G18.

FIG. 1

FIG. 2

EP 2 723 888 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005047544 A1
- US 2006269935 A1

**Non-patent literature cited in the description**

- **S.-I. HUNG et al.** Common risk allele in aromatic antiepileptic-drug induced Stevens-Johnson syndrome and toxic epidermal necrolysis in Han Chinese. *PHARMACOGENOMICS,* 01 January 2010, vol. 11 (3), ISSN 1462-2416, 349-356
- **WILLIAM KUDZI et al.** Characterisation of CYP2C8, CYP2C9 and CYP2C19 polymorphisms in a Ghanaian population. *BMC MEDICAL GENETICS,* 02 December 2009, vol. 10 (1), ISSN 1471-2350, 124
- **ROBERT T. KINOBE et al.** P450 2C18 Catalyzes the Metabolic Bioactivation of Phenytoin. *CHEMICAL RESEARCH IN TOXICOLOGY,* 01 December 2005, vol. 18 (12), ISSN 0893-228X, 1868-1875
- **LÓPEZ MARISOL et al.** Pharmacogenetics of the antiepileptic drugs phenytoin and lamotrigine. *DRUG METABOLISM AND DRUG INTERACTIONS,* May 2011, vol. 26 (1), ISSN 0792-5077, 5-12
- **JUNJI SARUWATARI et al.** Update on the genetic polymorphisms of drug-metabolizing enzymes in antiepileptic drug therapy. *PHARMACEUTICALS,* 20 August 2010, vol. 3 (8), ISSN 1424-8247, 2709-2732
- **J. A. GOLDSTEIN.** Clinical relevance of genetic polymorphisms in the human CYP2C subfamily. *BRITISH JOURNAL OF CLINICAL PHARMACOLOGY,* 01 October 2001, vol. 52 (4), ISSN 0306-5251, 349-355
- **LÖSCHER WOLFGANG et al.** The clinical impact of pharmacogenetics on the treatment of epilepsy. *EPILEPSIA,* 01 January 2009, vol. 50 (1), ISSN 0013-9580, 1-23
- **ULRICH KLOTZ.** The role of pharmacogenetics in the metabolism of antiepileptic drugs - Pharmacokinetic and therapeutic implications!. *CLINICAL PHARMACOKINETICS,* 2007, vol. 46 (4), ISSN 0312-5963, 271-279
- **BRANDOLESE et al.** Severe phenytoin intoxication in a subject homozygous for CYP2C9*3. *CLINICAL PHARMACOLOGY AND THERAPEUTICS,* 01 October 2001, vol. 70 (4), ISSN 0009-9236, 391-394

- **W.-H. CHUNG et al.** Genetic predisposition of life-threatening antiepileptic-induced skin reaction. *Expert Opin. Drug Saf.,* 2010, vol. 9 (1), 15-21
- **INGELMAN-SUNDBERG, M.** Pharmacogenomic biomarkers for prediction of severe adverse drug reactions. *N Engl J Med,* 2008, vol. 358, 637-639
- **BOHAN, K.H. et al.** Anticonvulsant hypersensitivity syndrome: implications for pharmaceutical care. *Pharmacotherapy,* 2007, vol. 27 (10), 1425-1439
- **ODANI, A. et al.** Genetic polymorphism of the CYP2C subfamily and its effect on the pharmacokinetics of phenytoin in Japanese patients with epilepsy. *Clin Pharmacol Ther,* 1997, vol. 62, 287-292
- **SHINTANI et al.** Genetic polymorphisms and functional characterization of the 5'-flanking region of the human CYP2C9 gene: In vitro and in vivo studies. *Clin Pharmacol Ther.,* 2001, vol. 70 (2), 175-182
- **LEE, A.Y. ; KIM, M.J. ; CHEY, W.Y. ; CHOI, J. ; KIM, B.G.** Genetic polymorphism of cytochrome P450 2C9 in diphenylhydantoin-induced cutaneous adverse drug reactions. *Eur J Clin Pharmacol,* 2004, vol. 60, 155-159
- **HUNG, S.I. et al.** Common risk allele in aromatic antiepileptic-drug induced Stevens-Johnson syndrome and toxic epidermal necrolysis in Han Chinese. *Pharmacogenomics,* 2010, vol. 11, 349-356
- Clinical classification of cases of toxic epidermal necrolysis, Stevens-Johnson syndrome, and erythema multiforme. **BASTUJI-GARIN S ; RZANY B ; STERN RS et al.** Arch Dermatol. 1993, vol. 129, 92-6
- **KARDAUN SH ; SIDOROFF A ; VALEYRIE-AL-LANORE L ; HALEVY S ; DAVIDOVICI BB ; MOCK-ENHAUPT M et al.** Variability in the clinical pattern of cutaneous side-effects of drugs with systemic symptoms: does a DRESS syndrome really exist?. *Br J Dermatol,* 2007, vol. 156, 609-11